# EUROPEAN PATENT APPLICATION

(11) **EP 2 703 815 A1**
(43) Date of publication of application: **05.03.2014**
(21) Application number: 12777358.8
(22) Date of filing: 23.04.2012
(51) Int. Cl.: G01N 33/53

(54) **METHOD FOR DETERMINING PROGNOSIS OF RENAL FAILURE**

(30) Priority: 25.04.2011 JP 2011096718
(71) Applicant: Kyowa Medex Co., Ltd., Tokyo 104-6004 (JP)
(72) Inventor: HAMANO, Takayuki, Osaka 5650871 (JP); ISAKA, Yoshitaka, Osaka 5650871 (JP); MATSUI, Isao, Osaka 5650871 (JP)
(74) Representative: Vossius & Partner
(86) International application number: PCT/JP2012/060818
(87) International publication number: WO 2012/147670

(57) **Abstract**

The present invention relates to a method for determining the prognosis of renal failure, which comprises measuring fibroblast growth factor-23 and 25-hydroxyvitamin D in a biological sample, and a kit for determining the prognosis of renal failure, which comprises a reagent for measuring fibroblast growth factor-23 and a reagent for measuring 25-hydroxyvitamin D.

The present invention provides a method for determining the prognosis of renal failure and a kit for determining the prognosis of renal failure, which are useful for deciding on a therapeutic strategy, such as selection of medication, introduction of a stricter diet therapy, and early introduction of dialysis treatment.

## Description

### Technical Field

The present invention relates to methods for determining the prognosis of renal failure and kits for determining the prognosis of renal failure.

### Background Art

Fibroblast growth factor-23 (hereinafter, referred to as FGF-23) is a member of the fibroblast growth factor (FGF) family and a polypeptide consisting of 251 amino acids, which is produced mainly in bone tissues and acts on the kidney to inhibit reabsorption of phosphorus in the renal tubules. In recent years, involvement of FGF-23 in diseases such as hypophosphatemic rickets, neoplastic osteomalacia, and renal failure has been suggested (see, Non-Patent Document 1). Measuring FGF-23 in the blood is recognized as being useful for monitoring the pathological conditions of these diseases, and thus, FGF-23 is drawing attention as a marker in recent years (Patent Document 1).

25-Hydroxyvitamin D (hereinafter, referred to as 25(OH)D) is a substance produced by hydroxylation in the liver of position 25 in the side chain of vitamin D which was absorbed into the body. In the body, this is further metabolized to 1α,25-dihydroxyvitamin D [1α,25(OH)₂D] or 24,25-dihydroxyvitamin D [24,25(OH)₂D] in the kidney by hydroxylation at position 1 or 24.

Vitamin D itself has little physiological activity, and is generally seldom measured since its blood concentration varies greatly due to metabolism, transfer to fat tissues, and such. In the body, vitamin D is quickly metabolized by 25-hydroxylase in the liver and is converted to 25(OH)D, which is then further metabolized in the kidney to the active-form 1α,25-dihydroxyvitamin D by hydroxylation at position1α by 1α-hydroxylase.

Active-form 1α,25-dihydroxyvitamin D promotes absorption of calcium and phosphorus from the small intestine, and promotes elution of bone minerals from the bones. Furthermore, it promotes resorption of calcium and phosphorus in the kidney and contributes to maintenance of homeostasis of calcium and phosphorus in a living body.

Vitamin D deficiency causes rickets in children and osteomalacia and osteoporosis in adults. Measurement of 25(OH)D is recognized as being useful for monitoring the pathological conditions of vitamin D deficiency and insufficiency (Non-Patent Document 2).

### [Prior Art Documents]

### [Patent Documents]

[Patent Document 1] WO 2003/057733

### [Non-Patent Documents]

[Non-Patent Document 1] Kidney and Metabolic Bone Diseases, vol. 15(4), p. 351-356 (2002)
[Non-Patent Document 2] Kidney Int. Vol. 55(6), p. 2169-2177 (1999)

### Summary of the Invention

### [Problems to be Solved by the Invention]

An objective of the present invention is to provide methods and kits for determining the prognosis of renal failure, which are effective for deciding on a therapeutic strategy for patients with renal failure.

### [Means for Solving the Problems]

The present inventors carried out dedicated studies to solve the above-described problems and found that the prognosis of renal failure can be determined by measuring FGF-23 and 25(OH)D in a sample and making an evaluation by combining those measured values, and completed the present invention. More specifically, the present invention relates to [1] to [6] below:
[1] a method for determining the prognosis of renal failure, wherein the method comprises measuring FGF-23 and 25(OH)D in a biological sample;
[2] the method of [1], wherein FGF-23 is measured by an immunological measurement method;
[3] the method of [1] or [2], wherein 25(OH)D is measured by an immunological measurement method;
[4] a kit for determining the prognosis of renal failure, which comprises a reagent for measuring FGF-23 and a reagent for measuring 25(OH)D;
[5] the kit of [4], wherein the reagent for measuring FGF-23 is a reagent comprising an antibody that binds to FGF-23; and
[6] the kit of [4] or [5], wherein the reagent for measuring 25(OH)D is a reagent comprising an antibody that binds to 25(OH)D.

### [Effects of the Invention]

The present invention enables selection of a group of renal failure patients with poor prognosis, and provides a method and a kit for determining the prognosis of renal failure, which are useful for deciding on a therapeutic strategy such as selection of medication, introduction of a stricter diet therapy and early introduction of dialysis treatment.

### Brief Description of the Drawings

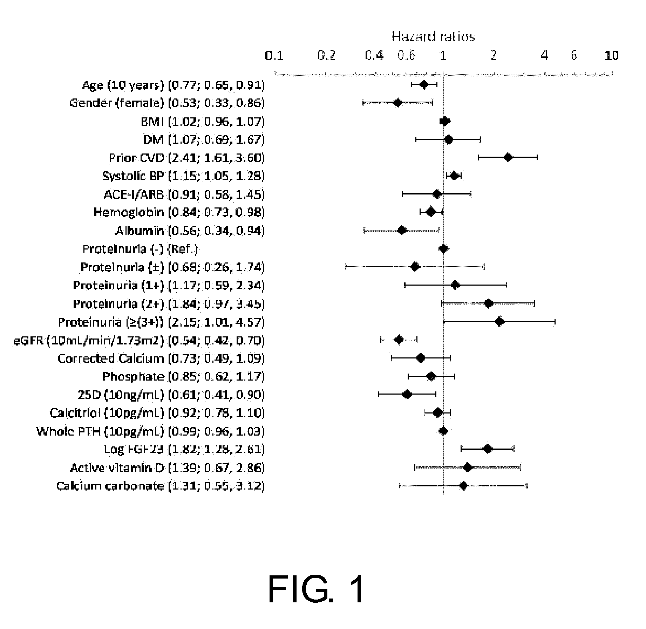

Fig. 1 shows an analysis diagram on the multivariate analysis on the risks of developing renal events (doubling of serum creatinine or introduction of dialysis treatment) using basic patient information and various measured values.

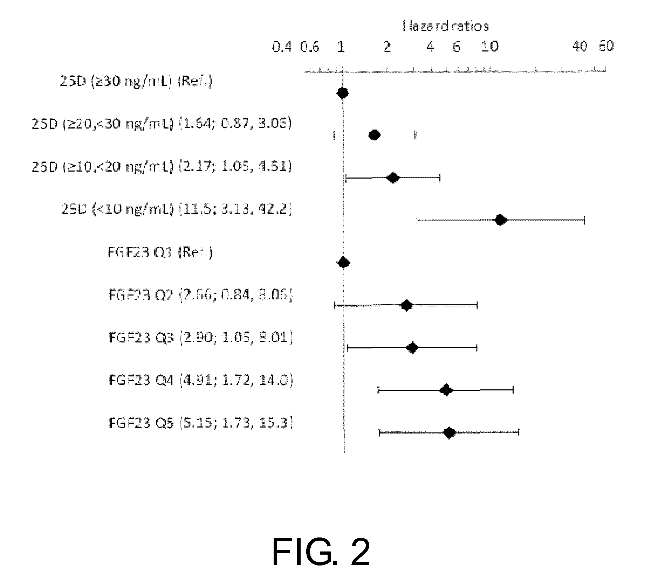

Fig. 2 shows an analysis diagram on the multivariate analysis on the risks of developing renal events (doubling of serum creatinine or introduction of dialysis treatment) in each of the patient groups that are based on the 25(OH)D concentration (four groups) and the patient groups that are based on the FGF-23 concentration (the five groups of Q1 to Q5).

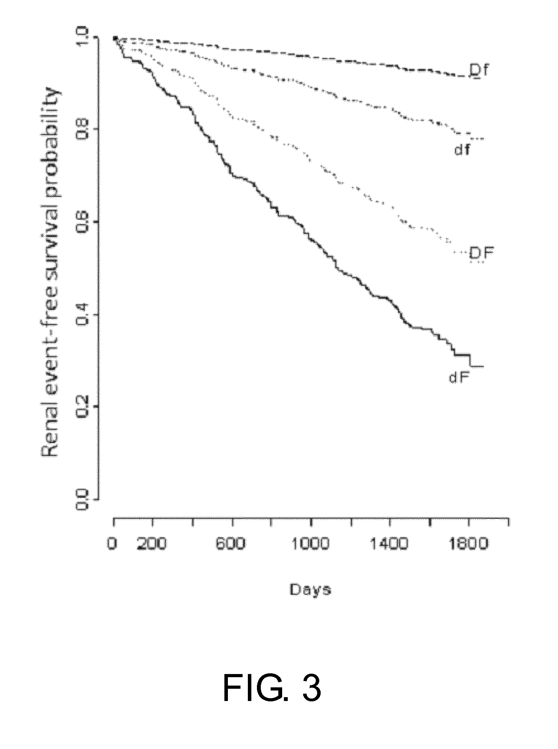

Fig. 3 shows Kaplan-Meier survival curves showing the probability of survival without renal events (doubling of serum creatinine or introduction of dialysis treatment) for approximately five years for the four groups of: the low 25(OH)D level and low FGF-23 level group (df group), the high 25(OH)D level and low FGF-23 level group (Df group), the low 25(OH)D level and high FGF-23 level group (dF group), and the high 25(OH)D level and high FGF-23 level group (DF group).

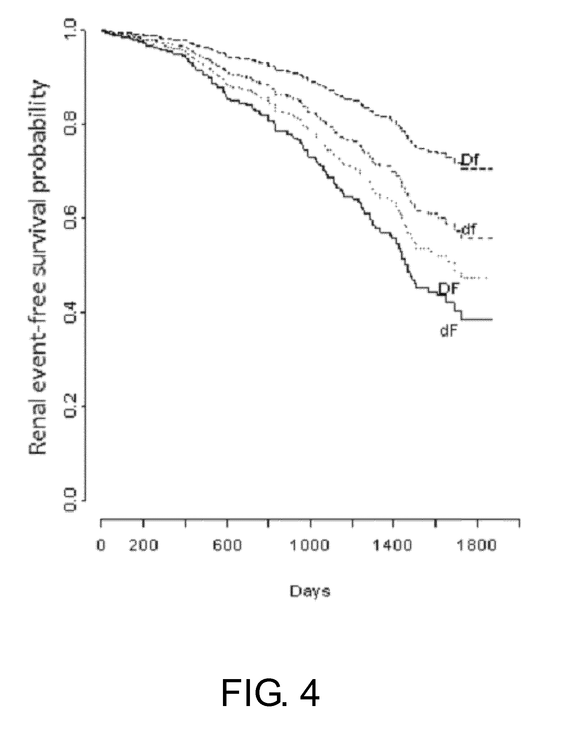

Fig. 4 shows Kaplan-Meier survival curves of the examination of Fig. 3 with further corrections for age, gender, diabetes, cardiovascular disease history, hypertension, hemoglobin, serum albumin, urinary protein, estimated glomerular filtration rate (eGFR), serum calcium, serum phosphorus, calcitriol [1,25-dihydroxyvitamin D₃], whole 1-84 parathyroid hormone (whole PTH), season of blood collection, administration of an angiotensin-converting enzyme (ACE) inhibitor/angiotensin receptor blocker (ARB), administration of active-form vitamin D, and administration of calcium.

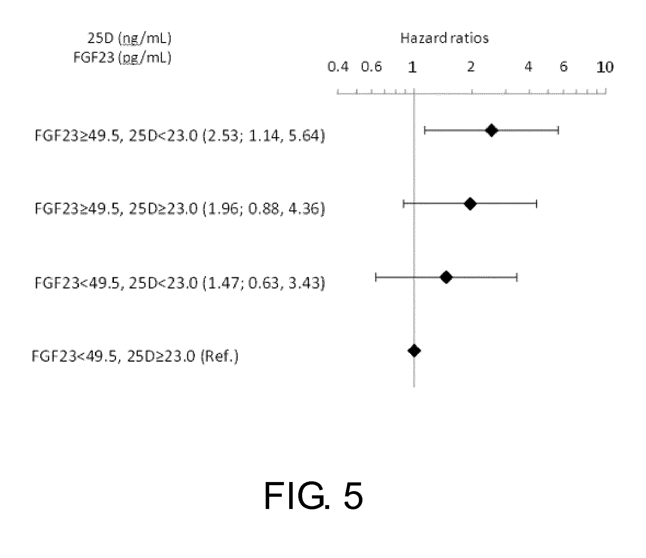

Fig. 5 shows an analysis diagram on the multivariate analysis on the risks of developing renal events (doubling of serum creatinine or introduction of dialysis treatment) in the four groups of 25(OH)D concentration and FGF-23 concentration combinations.

### Mode for Carrying Out the Invention

### <Method for determining the prognosis of renal failure>

The method of the present invention for determining the prognosis of renal failure comprises measuring FGF-23 and 25(OH)D in a biological sample. The method of the present invention for determining the prognosis of renal failure includes, for example, the following steps:
[1] measuring FGF-23 in a biological sample;
[2] determining the FGF-23 concentration in the biological sample by comparing the measured value obtained in step [1] with a calibration curve prepared in advance which shows the relationship between FGF-23 concentrations and measured values;
[3] measuring 25(OH)D in the biological sample;
[4] determining the 25(OH)D concentration in the biological sample by comparing the measured value obtained in step [3] with a calibration curve prepared in advance which shows the relationship between 25(OH)D concentrations and measured values; and
[5] based on the FGF-23 concentration determined in step [2] and the 25(OH)D concentration determined in step [4], determining that the prognosis of renal failure is poor when the FGF-23 concentration is not less than the median value determined based on the FGF-23 concentrations of all of the biological samples that were subjected to measurement and the 25(OH)D concentration is less than the median value determined based on the 25(OH)D concentrations of all of the biological samples that were subjected to measurement; and determining that the prognosis of renal failure is good when the FGF-23 concentration is less than the median FGF-23 value and the 25(OH)D concentration is not less than the median 25(OH)D value.

In step [5], when the median value of the FGF-23 concentrations and the median value of the 25(OH)D concentrations that were determined based on the FGF-23 concentrations and 25(OH)D concentrations of all of the biological samples subjected to measurement are, for example, 49.4 pg/mL and 23.0 ng/mL, respectively, the prognosis of renal failure of a subject whose FGF-23 concentration is 49.4 pg/mL or more and the 25(OH)D concentration is less than 23.0 ng/mL can be determined as being poor, and the prognosis of renal failure of a subject whose FGF-23 concentration is less than 49.4 pg/mL and the 25(OH)D concentration is 23.0 ng/mL or more can be determined as being good.

Here, the median value of the FGF-23 concentrations means the concentration of FGF-23 positioned exactly in the middle when FGF-23 concentrations determined for all the biological samples are arranged in order from the lowest value. Similarly, the median value of the 25(OH)D concentrations means the concentration of 25(OH)D positioned exactly in the middle when 25(OH)D concentrations determined for all the biological samples are arranged in order from the lowest value.

The biological sample of the present invention is not particularly limited as long as it is a sample that enables the measurement of FGF-23 and 25(OH)D, and examples include whole blood, serum, and plasma; and serum and plasma are preferred.

The method for measuring FGF-23 is not particularly limited as long as it is a method that enables a measurement of FGF-23, and examples include an immunological measurement method in which a measurement is carried out using FGF-23-binding antibodies. Examples of an immunological measurement method include any method that utilizes an antigen-antibody reaction, such as an immunoassay, an immunoblotting, an agglutination reaction, a complement fixation reaction, a hemolytic reaction, a precipitation reaction, a gold colloid method, a chromatography, and an immunostaining; and an immunoassay is preferred.

An immunoassay is a method of detecting or quantifying an antibody or an antigen using an antigen or an antibody labeled with various types of label, and depending on the method of labeling the antigen or antibody, examples include a radioimmunoassay (RIA), an enzyme immunoassay (EIA or ELISA), a chemiluminescent enzyme immunoassay (CLEIA or CLIA), a fluorescent immunoassay (FIA), a luminescent immunoassay, a physicochemical measurement method (TIA, LAPIA, and PCIA), and a flow cytometry; and a chemiluminescent enzyme immunoassay and such is preferred. In addition, an immunoassay may be a sandwich method or a competition method.

Measurement of FGF-23 in a biological sample using immunoassay can be carried out, for example, by the following method:
[1] reacting FGF-23 in a biological sample with a first antibody or a fragment thereof that binds to FGF-23 and that is immobilized onto a carrier and a labeled second antibody, in which a label is bound to a second antibody or a fragment thereof that binds to FGF-23, to form an immunocomplex comprising the first antibody, FGF-23, and the labeled second antibody on the carrier;
[2] measuring the label in the formed immunocomplex; and
[3] determining the FGF-23 concentration in the biological sample by comparing the measured value obtained in step [2] with a calibration curve prepared in advance which shows the relationship between FGF-23 concentrations and measured values.

A washing step may be inserted between step [1] and step [2] mentioned above.

The label is not particularly limited as long as it binds to the anti-FGF-23 antibody and enables measurement of FGF-23, and examples include peroxidase, alkaline phosphatase, β-galactosidase, a fluorescent substance, and a luminescent substance.

In case peroxidase is used as the label, FGF-23 can be measured using the later-described reagent for measuring the label. More specifically, FGF-23 can be measured by measuring the fluorescence, luminescence, and such generated by the reaction between the label (= peroxidase) in the immunocomplex and the reagent for measuring the label.

In case alkaline phosphatase is used as the label, FGF-23 can be measured using the later-described reagent for measuring the label. More specifically, FGF-23 can be measured by measuring the fluorescence, luminescence, and such generated by the reaction between the label (= alkaline phosphatase) in the immunocomplex and the reagent for measuring the label.

In case β-galactosidase is used as the label, FGF-23 can be measured using the later-described reagent for measuring the label. More specifically, FGF-23 can be measured by measuring the fluorescence, luminescence, and such generated by the reaction between the label (=β-galactosidase) in the immunocomplex and the reagent for measuring the label.

In case a fluorescent substance is used as the label, FGF-23 can be measured by measuring the fluorescence derived from the label (= the fluorescent substance) in the immunocomplex. Examples of the fluorescent substance include FITC (fluorescein isothiocyanate), RITC (rhodamine B-isothiocyanate), quantum dot (Science, 281, 2016-2018, 1998), phycobiliproteins such as phycoerythrin, GFP (Green fluorescent Protein), RFP (Red fluorescent Protein), YFP (Yellow fluorescent Protein), and BFP (Blue fluorescent Protein).

In case a luminescent substance is used as the label, FGF-23 can be measured by measuring the luminescence derived from the label (= the luminescent substance) in the immunocomplex. Examples of the luminescent substance include acridinium and derivatives thereof, a ruthenium complex compound, and lophine.

Furthermore, FGF-23 can be measured using a commercially available FGF-23-measuring kit. Examples of the commercially available FGF-23-measuring kit include the "FGF-23 assay reagent" (manufactured by KAINOS Laboratories, Inc.).

The method for measuring 25(OH)D is not particularly limited as long as it is a method that enables a measurement of 25(OH)D, and examples include an immunological measurement method in which a measurement is carried out using antibodies against 25(OH)D. The immunological measurement method include any method that utilizes an antigen-antibody reactions, such as an immunoassay, an immunoblotting, an agglutination reaction, a complement fixation reaction, a hemolytic reaction, a precipitation reaction, a gold colloid method, a chromatography, and an immunostaining. The method for measuring 25(OH)D is preferably, for example, an immunoassay method.

Examples of the immunoassay include the aforementioned measurement methods.

Measurement of 25(OH)D in a biological sample using an immunoassay can be carried out, for example, by the following method:
[1] reacting 25(OH)D in a biological sample with a first antibody or a fragment thereof that binds to 25(OH)D and that is immobilized onto a carrier and a labeled second antibody, in which a label is bound to a second antibody or a fragment thereof that binds to 25(OH)D, to form an immunocomplex comprising the first antibody, 25(OH)D, and the labeled second antibody on the carrier;
[2] measuring the label in the formed immunocomplex; and
[3] determining the 25(OH)D concentration in the biological sample by comparing the measured value obtained in step [2] with a calibration curve prepared in advance which shows the relationship between 25(OH)D concentrations and measured values.

A washing step may be inserted between step [1] and step [2] mentioned above.

The label is not particularly limited as long as it binds to the anti-25(OH)D antibody and enables measurement of 25(OH)D, and examples include the aforementioned labels. Furthermore, 25(OH)D can be measured using a commercially available 25(OH)D-measuring kit. Examples of the commercially available 25(OH)D-measuring kit include "LIAISON 25 OH Vitamin D TOTAL Assay" (manufactured by DiaSorin S.p.A.), and "25-OH Vitamin D, Direct ELISA Kit" (manufactured by Immundiagnostik AG).

### <Kit for determining the prognosis of renal failure>

The kit for determining the prognosis of renal failure of the present invention is a kit used for the method for determining the prognosis of renal failure of the present invention, and comprises a reagent for measuring FGF-23 and a reagent for measuring 25(OH)D. The kit of the present invention may also comprise a diluent for a biological sample, a reaction buffer, a washing solution, a reagent for detecting the label, and such. Furthermore, a device suitable for a measurement may be combined to produce the kit of the present invention.

### (1) Reagent for measuring FGF-23

The reagent for measuring FGF-23 is not particularly limited as long as it is a reagent that enables a measurement of FGF-23 in a biological sample, and examples include: (1) a reagent comprising a first antibody that binds to FGF-23 and that is immobilized onto a carrier and a labeled second antibody in which a label is bound to a second antibody that binds to FGF-23; (2) a reagent comprising an antibody that binds to FGF-23 and that is immobilized onto a carrier and a labeled competitive substance in which a label is bound to a competitive substance that competes with FGF-23; and (3) a reagent comprising a competitive substance that competes with FGF-23 and that is immobilized onto a carrier and a labeled antibody in which a label is bound to an FGF-23-binding antibody. As necessary, a diluent for a biological sample, a reaction buffer, a washing solution, a reagent for detecting the label, a standard material for FGF-23, and such may also be comprised in a reagent for measuring FGF-23.

The antibody that binds to FGF-23 (an anti-FGF-23 antibody) is not particularly limited as long as it is an antibody that binds to FGF-23, and examples include an anti-FGF-23 antibody from a goat, a rabbit, a rat, a mouse and such. The anti-FGF-23 antibody may be a polyclonal antibody or a monoclonal antibody. A fragment of anti-FGF-23 antibody may also be used. Examples of the antibody fragment include an antibody fragment with the Fc portions removed, such as Fab obtained by papain treatment of an antibody, F(ab')₂ obtained by pepsin treatment of an antibody, and Fab' obtained by pepsin treatment and reduction treatment of an antibody. In case of using the first antibody and the second antibody, the antigen-recognition site of the first antibody and the antigen-recognition site of the second antibody may be the same or different, and they are preferably different.

The competitive substance that competes with FGF-23 is not particularly limited as long as it is a substance that can compete with FGF-23, and examples include FGF-23 itself and a substance comprising a peptide corresponding to an antigen-recognition site of an anti-FGF-23 antibody.

The carrier is not particularly limited as long as it is a carrier that enables measurement of FGF-23, and examples include a plate, a latex, and magnetic particles. Examples of an immobilization of the anti-FGF-23 antibody and the competitive substance to the carrier include a physical method and a chemical method. Examples of a physical method include a method using a physical adsorption. Examples of a chemical method include a method using an avidin-biotin interaction and a method using a linker.

The diluent for a biological sample is not particularly limited as long as it is a diluent that enables measurement of FGF-23, and examples include an aqueous solution in which a surfactant, a protein, and such are contained in an aqueous medium. Examples of the aqueous medium include a deionized water, a distilled water, and a buffer. Examples of the buffer include a phosphate buffer and a Good's buffer. Examples of the Good's buffer include 2-morpholinoethanesulfonic acid (MES) buffer, bis(2-hydroxyethyl)iminotris(hydroxymethyl)methane (Bis-Tris) buffer, tris(hydroxymethyl)aminomethane (Tris) buffer, *N*-(2-acetoamido)imino diacetic acid (ADA) buffer, piperazine-*N,N*'-bis(2-ethanesulfonic acid) (PIPES) buffer, 2-[*N*-(2-acetamido)amino]ethanesulfonic acid (ACES) buffer, 3-morpholino-2-hydroxypropanesulfonic acid (MOPSO) buffer, 2-[*N,N*-bis(2-hydroxyethyl)amino]ethanesulfonic acid (BES) buffer, 3-morpholinopropanesulfonic acid (MOPS) buffer, 2-{*N*-[tris(hydroxymethyl)methyl]amino}ethanesulfonic acid (TES) buffer, *N*-(2-hydroxyethyl)-*N*'-(2-sulfoethyl)piperazine (HEPES) buffer, 3-[*N,N-*bis(2-hydroxyethyl)amino]-2-hydroxypropanesulfonic acid (DIPSO) buffer, 2-hydroxy-3-{[*N*-tris(hydroxymethyl)methyl]amino}propanesulfonic acid (TAPSO) buffer, piperazine-*N,N*'-bis(2-hydroxypropane-3-sulforiic acid) (POPSO) buffer, *N*-(2-hydroxyethyl)-N'-(2-hydroxy-3-sulfopropyl)piperazine (HEPPSO) buffer, *N*-(2-hydroxyethyl)-*N*'-(3-sulfopropyl)piperazine (EPPS) buffer, [*N*-tris(hydroxymethyl)methylglycine] (Tricine) buffer, [*N,N-*bis(2-hydroxyethyl)glycine] (Bicine) buffer, 3-[*N*-tris(hydroxymethyl)methyl]aminopropanesulfonic acid (TAPS) buffer, 2-(*N*-cyclohexylamino)ethanesulfonic acid (CHES) buffer, 3-(*N*-cyclohexylamino)-2-hydroxypropanesulfonic acid (CAPSO) buffer, and 3-(*N*-cyclohexylamino)propanesulfonic acid (CAPS) buffer.

The surfactant is not particularly limited as long as it is a surfactant that enables measurement of FGF-23, and examples include a nonionic surfactant, a cationic surfactant, an anionic surfactant, and an amphoteric surfactant. Examples of the proteins include bovine serum albumin (BSA), fetal bovine serum (FBS), casein, and BlockAce (manufactured by Dainippon Pharmaceutical Co., Ltd.).

The reaction buffer is not particularly limited as long as it is a buffer that enables measurement of FGF-23, and examples include the aforementioned buffer. A surfactant, a protein, a metal ion, a sugar, an antiseptic, and such may be included in the reaction buffer as necessary. Examples of the surfactant include the aforementioned surfactant. Examples of the protein include the aforementioned protein. Examples of the metal ion include magnesium ion, manganese ion, and zinc ion. Examples of the sugar include mannitol and sorbitol. Examples of the antiseptic include sodium azide, an antibiotic (streptomycin, penicillin, gentamicin, etc.), and BioAce.

The washing solution is not particularly limited as long as it is a washing solution that enables measurement of FGF-23, and examples include a phosphate buffered saline (10 mmol/L phosphate buffer containing 0.15 mol/L sodium chloride, pH7,2) (PBS). As necessary, the aforementioned surfactant, protein, antiseptic, and such may be included in the washing solution.

The reagent for measuring the label is a reagent for measuring the label in the immunocomplex produced by antigen-antibody reaction, and it is not particularly limited as long as it is a reagent for measuring the label that enables measurement of FGF-23. The label is not particularly limited as long as it is a label that binds to an anti-FGF-23 antibody and enables measurement of FGF-23, and examples include peroxidase, alkaline phosphatase, and β-galactosidase. As necessary, the aforementioned surfactant, protein, metal ion, sugar, antiseptic, and such may be included in the reagent for measuring the label.

In case peroxidase is used as the label, examples of the reagent for measuring the label include a reagent comprising hydrogen peroxide and a fluorescent substrate, and a reagent comprising hydrogen peroxide and a luminescent substrate. FGF-23 can be measured by measuring the fluorescence, luminescence, and such generated by the reaction between the label (= peroxidase) and the reagent for measuring the label. Examples of the fluorescent substrate include 4-hydroxyphenyl acetic acid, 3-(4-hydroxyphenyl)propionic acid, and coumarin. Examples of the luminescent substrate include the luminol compound and the lucigenin compound.

In case alkaline phosphatase is used as the label, examples of the reagent for measuring the label include a reagent comprising a substrate of alkaline phosphatase. FGF-23 can be measured by measuring the luminescence and such generated by the reaction between the label (= alkaline phosphatase) and the reagent for measuring the label. Examples of the substrate of alkaline phosphatase include
3-(2'-spiroadamantane)-4-methoxy-4-(3'-phosphoryloxy)phenyl-1,2-dioxetane disodium salt (AMPPD),
2-chloro-5-{4-methoxyspiro[1,2-dioxetane-3,2'-(5'-chloro)tricyclo[3.3.1.13,7]cane]-4-yl}phenyl phosphate disodium salt (CDP-Star™),
3-{4-methoxyspiro[1,2-dioxetane-3,2'-(5'-chloro)tricyclo[3.3.1.13,7]decane]-4-yl}phenylphosph ate disodium salt (CSPD™), [10-methyl-9(10H)-acridinylidene]phenoxymethylphosphate disodium salt (Lumigen™ APS-5), and
9-(4-chlorophenylthiophosphoryloxymethylidene)-10-methylacridone disodium salt.

In case β-galactosidase is used as the label, examples of the reagent for measuring the label include a reagent comprising a substrate of β-galactosidase. FGF-23 can be measured by measuring the fluorescence, luminescence, and such generated by the reaction between the label (= β-galactosidase) and the reagent for measuring the label. Examples of the substrate of β-galactosidase include 4-methylumbelliferyl-β-D-galactopyranoside, Galacton-Plus (manufactured by Applied Biosystems Inc.), and other similar compounds.

Examples of a standard material include FGF-23 obtained by a genetic engineering technique or obtained from a biological sample, and cells expressing FGF-23.

### (2) Reagent for measuring 25(OH)D

The reagent for measuring 25(OH)D is not particularly limited as long as it is a reagent that enables a measurement of 25(OH)D in a biological sample, and examples include: (1) a reagent comprising a first antibody that binds to 25(OH)D and that is immobilized onto a carrier and a labeled second antibody in which a label is bound to a second antibody that binds to 25(OH)D; (2) a reagent comprising an antibody that binds to 25(OH)D and that is immobilized onto a carrier and a labeled competitive substance in which a label is bound to a competitive substance that competes with 25(OH)D; and (3) a reagent comprising a competitive substance that competes with 25(OH)D and that is immobilized onto a carrier and a labeled antibody in which a label is bound to an antibody that binds to 25(OH)D. As necessary, a diluent for a biological sample, a reaction buffer, a washing solution, a reagent for detecting the label, a standard material for 25(OH)D, and such may also be comprised in the reagent for measuring 25(OH)D.

The antibody that binds to 25(OH)D (anti-25(OH)D antibodies) is not particularly limited as long as it is an antibody that binds to 25(OH)D, and examples include anti-25(OH)D antibody from a goat, a rabbit, a rat, a mouse, and such. The anti-25(OH)D antibody may be a polyclonal antibody or a monoclonal antibody. Fragment of anti-25(OH)D antibody may also be used. Examples of the antibody fragment include the aforementioned fragment. In case of using the first antibody and the second antibody, the antigen-recognition site of the first antibody and the antigen-recognition site of the second antibody may be the same or different, and they are preferably different.

The competitive substance that competes with 25(OH)D is not particularly limited as long as it is a substance that can compete with 25(OH)D, and examples include 25(OH)D itself and a substance having a partial structure of the 25(OH)D molecule that corresponds to the antigen-recognition site of an anti-25(OH)D antibody.

The carrier is not particularly limited as long as it is a carrier that enables measurement of 25(OH)D, and examples include the aforementioned carrier. Examples of immobilization of the anti-25(OH)D antibody and the competitive substance to the carrier include the aforementioned immobilization method.

The diluent for a biological sample is not particularly limited as long as it is a diluent that enables measurement of 25(OH)D, and examples include the aforementioned diluent.

The reaction buffer is not particularly limited as long as it is a buffer that enables measurement of 25(OH)D, and examples include the aforementioned buffer. As necessary, the aforementioned surfactant, protein, metal ion, sugar, antiseptic, and such may be included in the reaction buffer.

The washing solution is not particularly limited as long as it is a washing solution that enables measurement of 25(OH)D, and examples include the aforementioned washing solution.

The reagent for measuring the label is not particularly limited as long as it is a reagent for measuring the label that enables measurement of 25(OH)D. The label is not particularly limited as long as it is a label that binds to an anti-25(OH)D antibody and enables measurement of 25(OH)D, and examples include the aforementioned label. Examples of the reagent for measuring the label include the aforementioned reagent for measuring the label. As necessary, the aforementioned surfactant, protein, metal ion, sugar, antiseptic, and such may be included in the reagent for measuring the label.

Examples of the standard material include 25(OH)D prepared from a biological sample or chemically synthesized 25(OH)D. Commercially available 25(OH)D may also be used as the standard material.

Herein below, the present invention will be specifically described with reference to the Examples, but the Examples should not be construed as limiting the scope of the present invention.

### [Example 1]

### <Patients' background>

738 chronic kidney disease patients at the conservative phase were selected from patients with chronic kidney disease who are registered in the Osaka Vitamin D Study, and informed consents were obtained from them for this investigation. This investigation was approved by the ethics committee of Osaka University Hospital. Patients were registered from May 2005 to July 2007, and were monitored until July 2010.

### <Sample measurement>

Blood samples consisting of whole blood, serum, and plasma, as well as urine samples were collected at the time of patient registration. The blood samples were centrifuged within 30 minutes and the obtained serum were stored by freezing at -80°C until measurement. Creatinine, albumin, calcium, and inorganic phosphorus were measured and eGFR was calculated for each type of sample. eGFR was calculated according to the Japanese standard method based on inulin clearance.

Whole 1-84PTH was measured using the Whole PTH assay kit manufactured by Scantibodies Laboratory Inc. FGF-23 was measured using a kit manufactured by KAINOS Laboratories, Inc. Calcitriol was measured using the TFB 1,25-hydroxyvitamin D RIA kit manufactured by Immunodiagnostic Systems, PLC. 25(OH)D was measured using the ¹²⁵I RIA kit manufactured by DiaSorin S.p.A. Each of the measurements was carried out according to the conditions and methods described in the package insert of the respective companies.

### (1) Examination of hazard ratios for renal events (doubling of serum creatinine or introduction of dialysis treatment) and the 95% confidence intervals in multivariate analyses

Multivariate analyses were carried out on the risks for developing renal events using the patients' basic information and the various measured values. The results are shown in Fig. 1. Fig. 1 shows the relationship between the various risk factors and the hazard ratios, and the three numerical values of the various risk factors refer to, from the left, the hazard ratio, the lower limit of the 95% confidence interval, and the upper limit of the 95% confidence interval. Here, the hazard ratio is a value that indicates how many times the occurrence of a renal event is increased in the group with the specific risk factor when the group negative for urinary protein [Proteinuria (-)] is used as the control group. The 95% confidence interval is the range in which the population mean is included at a probability of 95%.

As is clear from Fig. 1, it was confirmed that, as the concentration of 25(OH)D [25D] increases by 10 ng/mL [25D (10 ng/mL)], as the age increases by ten years [Age (10 years)], and when the gender is female [Gender (female)], the hemoglobin level is high (Hemoglobin), and the eGFR level is high [eGFR (10 mL/min/1.73 m²)], development of nephropathy tends to be slow; and when FGF-23 level is high (Log FGF-23) and in the case of cardiovascular disease history (Prior CVD), hypertension (Systolic BP), and urinary protein level of 3+ or higher [Proteinuria ≥ (3+)], nephropathy tends to develop at an early stage.

### (2) Examination of the hazard ratios for renal events and the 95% confidence intervals in each of the 25(OH)D and FGF-23 groups

Four groups were made regarding the 25(OH)D [25D] concentration: less than 10 ng/mL; 10 ng/mL to less than 20 ng/mL; 20 ng/mL to less than 30 ng/mL; and 30 ng/mL or more. Five groups, Q1 to Q5 in the order from low FGF-23 concentration to high FGF-23 concentration, were made for the FGF-23 [FGF23] concentration as well based on the concentration range of 31.7 pg/mL to 80.5 pg/mL. The hazard ratios (HR) and the 95% confidence intervals (95% C.I.) were examined for each of the groups. The results are shown in Fig. 2. Fig. 2 shows the relationship between the various risk factors and the hazard ratios, and the three numerical values in parentheses for the various risk factors refer to, from the left, the hazard ratio, the lower limit of the 95% confidence interval, and the upper limit of the 95% confidence interval.

Regarding the 25(OH)D [25D] concentration, the hazard ratio for renal events showed a tendency to increase as the concentration shifted from high to low concentration, and the hazard ratio was highest (HR, 11.5) in the group with the lowest concentration (less than 10 ng/mL).

Regarding the FGF-23 [FGF23] concentration, the hazard ratio for renal events showed a tendency to increase in the high-concentration groups, and the hazard ratios were shown to increase as the FGF-23 concentration increased, in the order of: group Q2, group Q3, group Q4, and group Q5.

### (3) Examination of Kaplan-Meier survival curves for renal events in each of the groups of 25(OH)D and FGF-23 combinations

The median value of the 25(OH)D concentrations of all the subjects was 23.0 ng/mL, and the median value of the FGF-23 concentrations of all the subjects was 49.4 pg/mL. Therefore, 25(OH)D concentration of less than 23.0 ng/mL was defined as the low level group and 25(OH)D concentration of 23.0 ng/mL or more was defined as the high level group; and FGF-23 concentration of less than 49.4 pg/mL was defined as the low level group and FGF-23 concentration of 49.4 pg/mL or more was defined as the high level group. According to the each of the combinations, subjects were categorized into four groups: the low 25(OH)D concentration level and low FGF-23 concentration level group (df group); the high 25(OH)D concentration level and low FGF-23 concentration level group (Df group); the low 25(OH)D concentration level and high FGF-23 concentration level group (dF group); and the high 25(OH)D concentration level and high FGF-23 concentration level group (DF group). Their Kaplan-Meier survival curves for renal events (doubling of serum creatinine or introduction of dialysis treatment) for approximately five years were prepared. Here, a Kaplan-Meier survival curve shows, for each of the four groups, the probability of survival without development of renal events in relation to the number of days.

Fig. 3 shows the Kaplan-Meier survival curves prepared based on data before correction for age, gender, diabetes, cardiovascular disease history, hypertension, hemoglobin, serum albumin, urinary protein, eGFR, serum calcium, serum phosphorus, calcitriol, whole PTH, season of blood collection, administration of ACE inhibitor/ARB, administration of active-form vitamin D, and administration of calcium, and Fig. 4 shows the Kaplan-Meier survival curves prepared based on corrected data.

As Figs. 3 and 4 clearly show, it was confirmed both with the data before correction and the data after correction that the development of renal events significantly increased in the low 25(OH)D level and high FGF-23 level group (dF group).

### (4) Examination of the hazard ratios for renal events and the 95% confidence intervals in each of the groups of 25(OH)D and FGF-23 combinations

The hazard ratios for renal events and the 95% confidence intervals in the aforementioned four groups of 25(OH)D [25D] and FGF-23 [FGF23] combinations were examined. The results are shown in Fig. 5.

As shown in Fig. 5, the high 25(OH)D concentration level and low FGF-23 concentration level group (Df group) was used as the standard, and the hazard ratio (HR) and 95% confidence interval (95% C.I.) were 1.47 and 0.63-3.43, respectively, for the low 25(OH)D concentration level and low FGF-23 concentration level group (df group); the hazard ratio (HR) and 95% confidence interval (95% C.I.) were 1.96 and 0.88-4.36, respectively, for the high 25(OH)D concentration level and high FGF-23 concentration level group (DF group); and the hazard ratio (HR) and 95% confidence interval (95% C.I.) were 2.53 and 1.14-5.64, respectively, for the low 25(OH)D concentration level and high FGF-23 concentration level group (dF group). Therefore, the low 25(OH)D concentration level and high FGF-23 concentration level group (dF group) was confirmed to show the highest risk for renal events.

### Industrial Applicability

The present invention provides a method and a kit for determining the prognosis of renal failure, which are useful for deciding on a therapeutic strategy for patients with renal failure.

## Claims

1. A method for determining the prognosis of renal failure, wherein the method comprises measuring fibroblast growth factor-23 and 25-hydroxyvitamin D in a biological sample.

2. The method of claim 1, wherein fibroblast growth factor-23 is measured by an immunological measurement method.

3. The method of claim 1 or 2, wherein 25-hydroxyvitamin D is measured by an immunological measurement method.

4. A kit for determining the prognosis of renal failure, which comprises a reagent for measuring fibroblast growth factor-23 and a reagent for measuring 25-hydroxyvitamin D.

5. The kit of claim 4, wherein the reagent for measuring fibroblast growth factor-23 is a reagent comprising an antibody that binds to fibroblast growth factor-23.

6. The kit of claim 4 or 5, wherein the reagent for measuring 25-hydroxyvitamin D is a reagent comprising an antibody that binds to 25-hydroxyvitamin D.
